# EUROPÄISCHE PATENTSCHRIFT

(11) **EP 3 095 406 B1**
(45) Veröffentlichungstag und Bekanntmachung des Hinweises auf die Patenterteilung: **29.04.2020**
(21) Anmeldenummer: 15168061.8
(22) Anmeldetag: 19.05.2015
(51) Int. Cl.: A61B 18/16, A61F 7/02

(54) **NEUTRALELEKTRODENVORRICHTUNG ZUR APPLIKATION EINES HF-STROMS, ELEKTROCHIRURGISCHES SYSTEM MIT ENTSPRECHENDER NEUTRALELEKTRODENVORRICHTUNG UND VERFAHREN ZUR HERSTELLUNG EINER NEUTRALELEKTRODENVORRICHTUNG**
NEUTRAL ELECTRODE DEVICE FOR APPLYING A HF CURRENT, ELECTROSURGICAL SYSTEM WITH CORRESPONDING NEUTRAL ELECTRODE DEVICE AND METHOD FOR PRODUCING A NEUTRAL ELECTRODE DEVICE
DISPOSITIF D'ÉLECTRODE NEUTRE DESTINÉ À L'APPLICATION D'UNE PUISSANCE HF, SYSTÈME ÉLECTROCHIRURGICAL DOTÉ DU DISPOSITIF D'ÉLECTRODE NEUTRE CORRESPONDANT ET PROCÉDÉ DE FABRICATION D'UN DISPOSITIF D'ÉLECTRODE NEUTRE

(43) Veröffentlichungstag der Anmeldung: 23.11.2016
(73) Patentinhaber: Erbe Elektromedizin GmbH, 72072 Tübingen (DE)
(72) Erfinder: Schnitzler, Uwe, 72074 Tübingen (DE); Selig, Peter, 72379 Hechingen (DE)
(74) Vertreter: Bohnenberger, Johannes

(56) Entgegenhaltungen:
- EP-A1- 2 559 411
- DE-A1-102008 046 300
- DE-U1-202011 001 473
- US-A1- 2005 261 678
- US-A1- 2009 229 593

## Beschreibung

Die Erfindung betrifft eine Neutralelektrodenvorrichtung mit einem Latentwärmespeicher bzw. einem Phasenwechselmaterial ("PCM": Phase Change Material), ein System mit einer entsprechenden Neutralelektrodenvorrichtung und ein Verfahren zur Herstellung einer Neutralelektrodenvorrichtung.

Bei der Hochfrequenzchirurgie (HF-Chirurgie) wird Wechselstrom mit hoher Frequenz durch den menschlichen Körper geleitet, um Gewebe gezielt zu bearbeiten bzw. zu schneiden. Ein wesentlicher Vorteil gegenüber herkömmlichen Schneidetechniken mit dem Skalpell ist, dass gleichzeitig mit dem Schnitt eine Blutungsstillung durch Verschluss der betreffenden Gefäße erfolgen kann.

Häufig wird eine monopolare Technik angewandt. Dabei wird ein Pol der HF-Spannungsquelle über eine möglichst große Fläche mit dem Patienten verbunden. Die Elektrode nennt man Neutralelektrode. Der andere Pol (Aktivelektrode) befindet sich an einem chirurgischen Instrument. Der Strom fließt von der Aktivelektrode zur Neutralelektrode. In unmittelbarer Nähe der Aktivelektrode ist die Stromdichte am höchsten. Hier findet eine Koagulation oder Durchtrennung des Gewebes statt.

Bei Neutralelektroden muss darauf geachtet werden, dass zwischen Haut und anliegender Elektrode kein zu hoher Übergangswiderstand auftritt. Dies würde zu einer starken Erwärmung des biologischen Gewebes, mitunter zu Verbrennungen führen. In letzter Zeit stellt sich das Problem, dass immer mehr Verfahren entwickelt wurden, bei denen relativ große HF-Ströme über längere Zeitdauer appliziert werden. Das Risiko einer Verbrennung an der Neutralelektrode wird somit erhöht. Hierbei sei noch angemerkt, dass durch die physikalischen Gegebenheiten an den Randbereichen der Neutralelektrode die maximale Erwärmung auftritt. Daher ist in diesen Randbereichen das Risiko einer Verbrennung besonders hoch.

Um eine ungewollte Schädigung des Gewebes zu vermeiden, werden entsprechend großflächige Neutralelektroden verwendet, die dazu beitragen, die Stromdichte in unmittelbarer Nachbarschaft der Neutralelektrode zu verringern. Darüber hinaus gibt es Überwachungseinrichtungen, die den Zweck haben, eine teilweise Ablösung der Neutralelektrode zu erkennen und auf dieses Ereignis entsprechend zu reagieren.

Aktuelle Normen schreiben Untersuchungen vor, die einen Temperaturanstieg an der Neutralelektrode bei der Applikation eines gewissen Stroms über einen vorgegebenen Zeitraum auf einen Maximalwert begrenzen.

Aus der DE 10 2008 046 300 A1 ist eine Neutralelektrodenvorrichtung bekannt, die Phasenwechselmaterial zur Aufnahme von Wärme und somit zur Abfederung von thermischen Spitzen verwendet. Ein Problem dieser Neutralelektrodenvorrichtung besteht darin, dass diese relativ kleinflächig ausgebildet werden muss, um einen sicheren Sitz am Gewebe zu gewährleisten.

Es sei noch auf die US 6,183,855 B1 verwiesen, die allgemein die Verwendung von Phasenwechselmaterial in Kleidungsstücken offenbart.

In der US 2009/229593 A1 wird eine Vorrichtung mit Phasenwechselmaterial beschrieben, die Wärmeenergie speichern und zu einem späteren Zeitpunkt abgeben soll. Das in dieser Druckschrift beschriebene Phasenwechselmaterial ist ein Pulver oder eine Flüssigkeit, die vor äußeren Einflüssen geschützt werden sollen. Hierfür schlägt die Druckschrift vor, aus einer Folie einzelne Kammern zu erzeugen, in die das Phasenwechselmaterial eingebracht wird.

Ausgehend von der DE 10 2008 046 300 A1 ist es Aufgabe der vorliegenden Erfindung, eine verbesserte Neutralelektrodenvorrichtung anzugeben. Insbesondere soll die Fähigkeit, Wärme aufzunehmen, bei einem guten Anlageverhalten (Kontakt der Neutralelektrodenvorrichtung zum Gewebe) verbessert werden.

Des Weiteren soll ein entsprechendes elektrochirurgisches System sowie ein Verfahren zur Herstellung einer Neutralelektrodenvorrichtung angegeben werden. Die Aufgabe wird durch eine Neutralelektrodenvorrichtung gemäß dem Anspruch 1 bzw. einem System gemäß dem Anspruch 10 bzw. ein Verfahren zur Herstellung gemäß dem Anspruch 11 gelöst.

Das Phasenwechselmaterial ist für eine bessere Flexibilität in Blöcke einer Matrix gegossen, wobei diese zumindest abschnittsweise zueinander Abstände aufweisen. Durch die Abstände bzw. durch die Abstandsspalten wird eine höhere Flexibilität im Vergleich zu einer flächig beschichteten Neutralelektrodenvorrichtung erreicht. Dadurch wird eine Neutralelektrodenvorrichtung möglich, die bei Patienten an entsprechend dafür vorgesehenen Oberflächen, beispielsweise am Oberschenkel, angebracht werden kann. Diese Oberflächen weisen bei verschiedenen Patienten durchaus unterschiedliche Formen und Rundungen auf. Durch die Abstände zwischen den Blöcken wird eine Flexibilität erreicht, wodurch sich die Neutralelektrodenvorrichtung bei nahezu allen Patienten anwendungsgerecht anbringen lässt. Vorzugsweise werden die Blöcke auf einer zweiten Seite der Trägerstruktur der Neutralelektrodenvorrichtung angeordnet. Hierbei handelt es sich vorzugsweise um die Seite der Trägerstruktur der Neutralelektrodenvorrichtung, die bei einer Anbringung am Patienten dem Gewebe abgewandt ist (gewebeabgewandte Seite).

Die Trägerstruktur kann eine Vielzahl von Elektroden umfassen. In Abhängigkeit von der Definition der Trägerstruktur können auch auf die erste Seite der Trägerstruktur Elektroden aufgebracht sein. Vorzugsweise sind die verwendeten Elektroden dünnschichtig und/oder aus einer Aluminiumlegierung hergestellt.

In einer Ausführungsform weist die Neutralelektrodenvorrichtung eine leitende Substanz, insbesondere aus der Gruppe der viskoelastischen Fluide auf. Hierbei kann es sich um Hydrogel handeln, das den Kontaktschluss zwischen der mindestens einen Elektrode und dem Gewebe verbessert. Vorzugsweise ist die leitende Substanz auf der ersten - gewebezugewandten - Seite der Trägerstruktur vorgesehen.

Zumindest einige der Blöcke können als geometrische Körper ausgebildet sein. Vorzugsweise kann es sich hierbei um Polyeder handeln. Die geometrischen Körper lassen sich in einfacher Weise ausbilden und derart anordnen, dass sie eine hohe Flexibilität der Neutralelektrodenvorrichtung gewährleisten.

Die Blöcke oder zumindest einige der Blöcke können sich in Richtung von der Trägerstruktur weg verjüngen. Die Abstände zwischen den Blöcken sind also nicht zwingend konstant. Es ist möglich, dass sich die Abstände in Richtung der Füße der Blöcke reduzieren. Erfindungsgemäß können sich die Blöcke im Bereich ihrer Füße berühren und miteinander verbunden sein. In den Bereichen, die weiter von der Trägerstruktur entfernt sind, entstehen die bereits genannten Abstandsspalten.

Die Verbreiterung der Blöcke in Richtung auf die Trägerstruktur zu hat den Vorteil, dass eine möglichst großflächige Energieaufnahme erzielt werden kann, wobei die Flexibilität kaum eingeschränkt wird. In einer Ausführungsform erstrecken sich die von den Blöcken begrenzten Abstandsspalten entlang von Geraden. Es kann sich also beispielsweise ein ganzes Netz von Geraden bzw. Abstandsspalten bilden. In einer Ausführungsform trifft sich eine Teilmenge dieser Geraden rechtwinklig oder liegt parallel nebeneinander.

In einer (anderen) Ausführungsform nehmen zumindest einige der sich schneidenden Geraden, entlang derer die Abstandsspalten ausgebildet sind, einen Winkel ein. Der jeweils gemessene Minimalwinkel kann sich in einem Bereich von 30-90°, vorzugsweise 50-90° erstrecken. Insofern ergibt sich eine regelmäßige Anordnung der Blöcke, die eine hohe Flexibilität gewährleistet.

Die Blöcke können verschiedene Formen aufweisen. Sie können beispielsweise als Rechtecke, Strukturen mit trapezartigen Querschnitten oder tropfenartig ausgebildet sein. Vorzugsweise sind die Füße der Blöcke mit der Trägerstruktur verbunden. Die Blöcke können eine Höhe von maximal 1 cm oder 8 mm oder 6 mm haben. Vorzugsweise haben sie eine Maximalhöhe von 4 mm, gemessen von einer Ebene, die die Trägerstruktur, insbesondere deren zweite Seite, aufspannt. In einem Ausführungsbeispiel hat ein Block ein Maximalvolumen von 1 cm³, vorzugsweise von 0,5 cm³.

In einer Ausführungsform hat eine Vielzahl der Blöcke eine identische Form. Die Blöcke können zumindest teilweise mit konstantem Abstand zueinander und/oder konstantem Versatz zueinander auf der Trägerstruktur angeordnet sein.

Zumindest einige der Blöcke können thermochrome Farbstoffe und/oder Farbpigmente (z.B. 0,1 bis 4, insbesondere 0,5 bis 2,5, prozentualer Gewichtanteil) umfassen. Es ist möglich, thermochrome Farbe zu nutzen, um einen Schmelzstatus des Phasenübergangsmaterials sichtbar zu machen. Insofern dient das Phasenübergangsmaterial mit den thermochromen Farbstoffen nicht nur zur Absorption von Wärme sondern auch zur Anzeige einer Gefahrensituation oder eines Nutzungszustands (z.B. Die Speicherkapazität des Latentwärmespeichers ist zu 50% verbraucht).

In einer erfindungsgemäßen Ausführungsform sind zumindest einige der Blöcke mehrschichtig ausgebildet, wobei sich die Schichten vorzugsweise parallel zur Trägerstruktur erstrecken. Beispielsweise können eine erste untere Schicht mit einem ersten Phasenwechselmaterial und eine zweite vorzugsweise obere Schicht mit einem Phasenwechselmaterial vorgesehen sein. Das erste Phasenwechselmaterial kann sich hinsichtlich des Schmelzpunktes von dem zweiten Phasenwechselmaterial unterscheiden. Beispielsweise kann der Schmelzpunkt des ersten Phasenwechselmaterials 5 und/oder 10 und/oder 20% höher sein als der Schmelzpunkt des zweiten Phasenwechselmaterials. Es ist denkbar, weitere Phasenwechselmaterialien bzw. Schichten von Phasenwechselmaterialien vorzusehen, die sich jeweils hinsichtlich des Schmelzpunkts unterscheiden. In einer bevorzugten Ausführungsform sind die Blöcke derart aus Phasenwechselmaterialien hergestellt, dass der Schmelzpunkt kontinuierlich oder in diskreten Schritten mit zunehmender Distanz zur Trägerstruktur abnimmt. Beispielsweise kann eine unterste Schicht bei ca. 30°C und darüber liegende Schichten bei 28° und 25°C schmelzen. Dies führt zu einem verbesserten Wärmetransport weg von den Elektroden und/oder dem Gewebe. Alternativ oder zusätzlich können die Blöcke derart ausgestaltet sein, dass sie an unterschiedlichen Positionen im Schichtaufbau eine sich unterscheidende Wärmeleitfähigkeit besitzen.

Allgemein kann erfindungsgemäß Phasenübergangsmaterial verwendet werden, dessen Schmelzpunkt im Bereich zwischen 20° und 40°, vorzugsweise 25° und 30° liegt.

Die eingangs genannte Aufgabe wird des Weiteren durch ein System, insbesondere ein elektrochirurgisches System zur Koagulation und/oder zum Schneiden von Gewebe gelöst. Das System umfasst vorzugsweise:
- eine Neutralelektrodenvorrichtung;
- mindestens ein elektrochirurgisches, insbesondere monopolares Instrument;
- einen HF-Generator, der mit der Neutralelektrodenvorrichtung und dem mindestens einem Instrument verbunden ist.

In einer Ausführungsform ist die Neutralelektrodenvorrichtung so ausgebildet, wie dies in einem der vorhergehenden Ausführungsformen erläutert wurde. Es ergeben sich ähnliche Vorteile, wie diese bereits in Verbindung mit der Neutralelektrodenvorrichtung benannt wurden.

Die eingangs genannte Aufgabe wird des Weiteren durch ein Verfahren zur Herstellung einer Neutralelektrodenvorrichtung gemäß Anspruch 11 gelöst.

In einer Ausführungsform weist die Neutralelektrodenvorrichtung einige oder alle der vorab beschriebenen Merkmale auf.

Auch hinsichtlich der Vorrichtung ergeben sich ähnliche Vorteile, wie diese in Verbindung mit der Neutralelektrodenvorrichtung erläutert wurden.

Durch die Verwendung der nach unten hin offenen Gussform lässt sich das Phasenübergangsmaterial schnell und effizient aufbringen.

Die Faserstruktur ist besonders geeignet, einen widerstandsfähigen Stoffschluss zu den Blöcken herzustellen.

In einer Ausführungsform werden in die Gussform nacheinander mehrere sich insbesondere anhand des Schmelzpunkts unterscheidende Phasenwechselmaterialien eingebracht. Dies führt dazu, dass sich der bereits erläuterte Schichtaufbau bildet, der wiederum die Wärmeleitfähigkeit bzw. den Wärmetransport verbessert.

Mindestens eines der Phasenwechselmaterialien wird in einer Polymerstruktur verarbeitet. Die Polymerstruktur ist schwammartig ausgebildet. Die Polymerstruktur kann zu einer besseren Verarbeitung dienen. Des Weiteren kann sie dafür sorgen, dass die aufgebrachten Blöcke ihre Form behalten, auch wenn das Phasenwechselmaterial teilweise oder gänzlich während der Anwendung schmilzt. Insofern wird die Flexibilität der erfindungsgemäßen Neutralelektrodenvorrichtung auch nach dem erneuten Aushärten des Phasenwechselmaterials aufrechterhalten.

Die Trägerstruktur kann Gewebe, Gelege, Vliese oder Gewirke umfassen. Vorzugsweise werden grobmaschige Gewebe, Gelege, Vliese oder Gewirke verwendet, die ein zumindest teilweises Ein- und/oder Durchdringen des Phasenwechselmaterials in einen zumindest teilweise verflüssigten Zustand ermöglichen. Dies vereinfacht die Herstellung und verbessert die Wärmeleitfähigkeit der Neutralelektrodenvorrichtung. Das Gewebe, Gelege, Vliese oder Gewirke kann beispielsweise eine geringe Fadendichte in Längs- und/oder Querrichtung aufweisen, z.B. maximal 30 oder 20 oder 10 Fäden pro Zentimeter.

Weitere vorteilhafte Ausgestaltungen ergeben sich anhand der Unteransprüche. Nachfolgend wird die Erfindung anhand einiger Zeichnungen erläutert.

Hierbei zeigen:
- Fig. 1: ein elektrochirurgisches System mit einem monopolaren elektrochirurgischen Instrument zum Schneiden und/oder Koagulieren von Gewebe sowie einer Neutralelektrodenvorrichtung;
- Fig. 2: eine schematische Detailansicht der Neutralelektrodenvorrichtung gemäß Fig. 1 mit einer Vielzahl von PCM-Blöcken, die auf einer Trägerstruktur angeordnet sind;
- Fig. 3: einen exemplarischen Schnitt durch die Neutralelektrodenvorrichtung, insbesondere einen PCM-Block, der Fig. 2;
- Fig. 4: eine Seitenansicht einer weiteren Neutralelektrodenvorrichtung mit trapezförmigen PCM-Blöcken;
- Fig. 5: ein weiteres Ausführungsbeispiel einer Neutralelektrodenvorrichtung mit zylinderförmigen PCM-Blöcken;
- Fig. 6: einen Schnitt durch eine weitere Neutralelektrodenvorrichtung, wobei die PCM-Blöcke mehrschichtig ausgebildet sind;
- Fig. 7: eine Draufsicht auf eine exemplarische Gussform zur Herstellung einer Neutralelektrodenvorrichtung gemäß Fig. 1.

In der nachfolgenden Beschreibung werden für gleiche und gleichwirkende Teile dieselben Bezugsziffern verwendet.

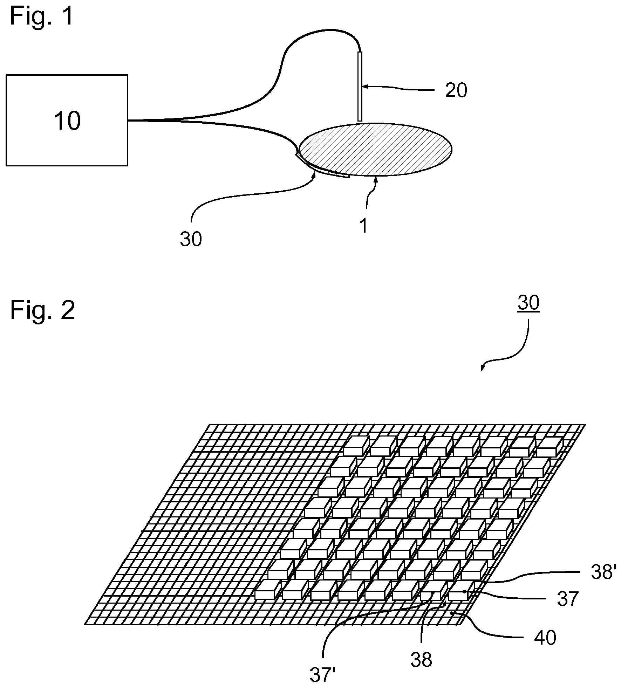

Fig. 1 zeigt ein elektrochirurgisches System. Dieses umfasst einen HF-Generator 10, ein monopolares Instrument 20 und eine Neutralelektrodenvorrichtung 30. Bei einer Applikation von HF-Strom liegt eine Spannung zwischen dem monopolaren Instrument 20 und der Neutralelektrodenvorrichtung 30 an. Der HF-Behandlungsstrom durchfließt biologisches Gewebe eines zu behandelnden Körpers, im vorliegenden Fall einen Torso 1. In unmittelbarer Nähe zu dem monopolaren Instrument 20 ist die Stromdichte derart hoch, dass das anliegende Gewebe koaguliert oder durchtrennt wird.

Um Verbrennungen an der Neutralelektrodenvorrichtung 30 zu vermeiden, soll erfindungsgemäß ein Latentwärmespeicher vorgesehen werden. Die Fig. 2 zeigt eine Detailansicht einer entsprechenden Neutralelektrodenvorrichtung 30, wobei diese eine Trägerstruktur 40 und eine Vielzahl von auf der Trägerstruktur 40 angeordneten PCM-Blöcken 37, 37' umfasst.

Die PCM-Blöcke 37, 37' sind regelmäßig, nämlich in einer Matrixanordnung, angeordnet. Um eine möglichst hohe Flexibilität der Neutralelektrodenvorrichtung 30 zu gewährleisten, sind die PCM-Blöcke 37, 37' voneinander beabstandet. Es gibt also Gräben zwischen den einzelnen PCM-Blöcken 37, 37', die in einer Längsanordnung bzw. Queranordnung Abstandsspalten 38 bzw. 38' ausbilden. In dem gezeigten Ausführungsbeispiel verlaufen die Abstandsspalten 38, 38' zueinander senkrecht oder parallel. Sie bilden ein Gitternetz von Abstandsspalten 38, 38'.

In dem Ausführungsbeispiel haben die PCM-Blöcke 37, 37' eine im Wesentlichen quadratische Grundfläche und eine Höhe von ca. 3 mm (gemessen von der Oberfläche der Trägerstruktur 40 bis zur äußeren Oberfläche der PCM-Blöcke 37, 37').

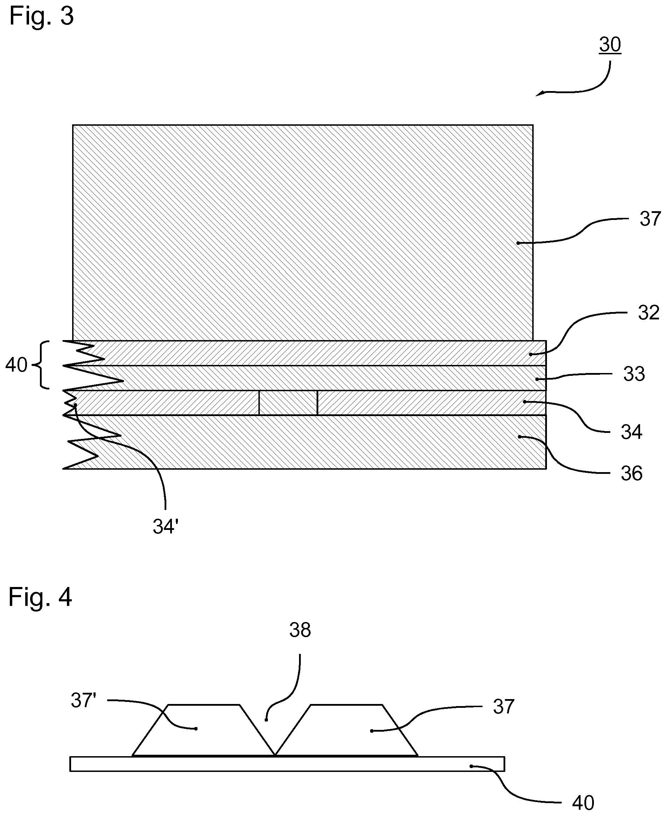

Fig. 3 zeigt einen schematischen Schnitt durch den Randbereich der Neutralelektrodenvorrichtung 30 aus Fig. 2. Wesentliche Komponenten der Neutralelektrodenvorrichtung 30 gemäß dem Schnitt sind die Trägerstruktur 40 mit ihrer oberen (gewebeabgewandten) und ihrer unteren (gewebezugewandten) Seite sowie der PCM-Block 37. Dieser PCM-Block 37 ist auf der oberen Seite der Trägerstruktur 40 angeordnet und befestigt. An der unteren Seite der Trägerstruktur 40 befinden sich mehrere Elektroden 34, 34', die voneinander beabstandet angeordnet sind. Eine Hydrogelschicht 36 bedeckt die Elektroden 34, 34' und bildet im angeordneten Zustand den unmittelbaren Kontaktschluss zu dem Gewebe, beispielsweise der Haut eines Patienten.

Die Trägerstruktur 40 umfasst ein Trägervlies 32 sowie einen PET-Träger 33. In dem beschriebenen Ausführungsbeispiel ist der PET-Träger 33 mit dem Trägervlies 32 verklebt. Der PET-Träger 33 kann eine Dicke von 20-80 µm haben. Im gezeigten Ausführungsbeispiel beläuft sich dessen Dicke auf 50 µm. Der PET-Träger 33 ist für die mechanische Stabilität der Neutralelektrodenvorrichtung 30 nützlich und wird auf der unteren Seite mit einem PET-Anteil der Elektroden 34, 34' und auf der anderen Seite mit dem Trägervlies 32 verbunden. Bei dem Trägervlies 32 kann es sich um ein Polyestervlies handeln. Die Klebeverbindung zum PET-Träger 32 kann über einen biokompatiblen Klebestoff hergestellt werden.

In dem beschriebenen Ausführungsbeispiel umfasst die Trägerstruktur 40 den PET-Träger 33 und das Trägervlies 32. Erfindungsgemäß kann die Trägerstruktur 40 auch derart definiert sein, dass diese weiterhin die Elektroden 34, 34' und ggf. die Schicht aus Hydrogel 36 aufweist.

Die PCM-Blöcke 37,37' umfassen eine schwammartige Polymerstruktur, die der besseren Verarbeitung dient und dafür sorgt, dass die aufgebrachte Struktur der PCM-Blöcke 37, 37' ihre Form behält, auch wenn das PCM schmilzt.

Fig. 4 zeigt schematisch eine Seitenansicht einer weiteren Neutralelektrodenvorrichtung 30. Auch hier sind PCM-Blöcke 37, 37' auf einer Trägerstruktur 40 angeordnet. Die Füße der Blöcke 37, 37' sind wie auch im vorhergehenden Ausführungsbeispiel mit der Trägerstruktur 40 verbunden. Mit zunehmender Distanz zu der Trägerstruktur 40 verjüngen sich die PCM-Blöcke 37. In dem gezeigten Ausführungsbeispiel haben die Blöcke in ihrem Längsschnitt und in ihrem Querschnitt eine trapezförmige Form. Es ist denkbar, die PCM-Blöcke 37, 37' derart auszubilden, dass eine entsprechende trapezförmige Ausgestaltung nur in einem der beiden Schnitte auftritt. Erfindungsgemäß können sich die Füße der PCM-Blöcke 37, 37' in diesem Ausführungsbeispiel berühren oder geringfügig voneinander beabstandet sein. Mit zunehmender Distanz zur Trägerstruktur 40 nimmt der Abstand zwischen den Seitenwänden der PCM-Blöcke 37, 37' zu. Es bilden sich Abstandsspalten 38, 38', beispielsweise die in Fig. 4 gezeigte Abstandsspalte 38, die in Längsrichtung verläuft.

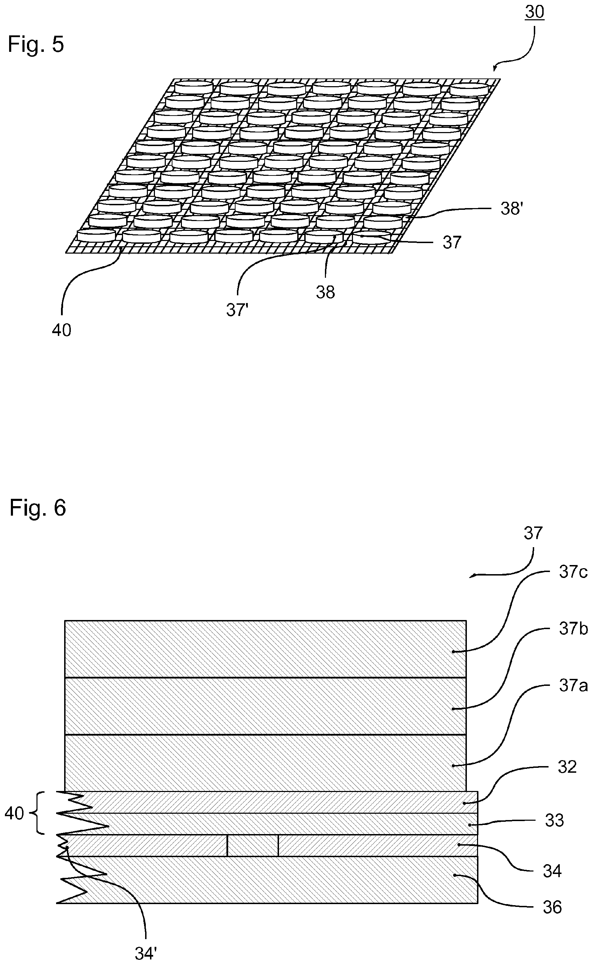

Fig. 5 zeigt eine weitere Ausführungsform der erfindungsgemäßen Neutralelektrodenvorrichtung 30. Hier sind die Blöcke 37, 37' als senkrechte oder gerade Kreiszylinder ausgebildet und ebenso wie bei den zuvor beschriebenen Ausführungsbeispielen auf einer Trägerstruktur 40 befestigt. Auch bei diesem Ausführungsbeispiel erfolgt die Anordnung in einer Art Matrix. Erfindungsgemäß können die PCM-Blöcke 37, 37' auch mit einem Versatz zueinander angeordnet werden. Beispielsweise ist es denkbar, das Ausführungsbeispiel gemäß Fig. 5 oder gemäß Fig. 2 derart abzuwandeln, dass eine zweite Zeile von PCM-Blöcken um ca. 3 mm zu einer ersten Zeile von PCM-Blöcken versetzt angeordnet ist. In diesem Ausführungsbeispiel schneiden sich einige der Abstandsspalten 38, 38' unter einem stumpfen bzw. spitzen Winkel.

Fig. 6 entspricht in einigen Merkmalen der Fig. 3. Jedoch hat in dem gezeigten Ausführungsbeispiel der PCM-Block 37 einen mehrschichtigen Aufbau. Von unten (gewebenah) nach oben (gewebefern) umfasst der PCM-Block 37 eine erste PCM-Schicht 37a, eine zweite PCM-Schicht 37b und eine dritte PCM-Schicht 37c. Erfindungsgemäß kann bei diesem mehrschichtigen Aufbau des PCM-Blocks 37 das jeweils verwendete PCM unterschiedliche Schmelzpunkte haben.

Beispielsweise kann die erste PCM-Schicht 37a derart konfiguriert sein, dass diese bei 30°C schmilzt. Die zweite PCM-Schicht 37b und die dritte PCM-Schicht 37c können derart konfiguriert sein, dass diese bei 28°C bzw. 25°C schmelzen. Die dritte PCM-Schicht schmilzt also zuerst und entzieht der darunterliegenden Schicht Wärme. Dies führt zu einem besseren Wärmetransport weg von dem Gewebe an dem die Neutralelektrodenvorrichtung angelegt ist. Erfindungsgemäß können mehr oder weniger Schichten vorgesehen sein. Auch muss der Schichtübergang zwischen den einzelnen PCM-Schichten 37a, 37b, 37c nicht derart diskret und/oder plan sein, wie dies in der Fig. 6 gezeigt ist. Die einzelnen PCM-Schichten 37a, 37b, 37c können sich in ihren Grenzbereichen durchmischen und/oder Grenzschichten ausbilden, die Wölbungen und Krater aufweisen. In dem beschriebenen Ausführungsbeispiel ist es ein wesentlicher Aspekt, dass es mindestens zwei Punkte innerhalb des PCM-Blocks 37 gibt, an denen das verwendete PCM einen unterschiedlichen Schmelzpunkt hat. Vorzugsweise ist hierbei der Schmelzpunkt des Punkts, der weiter von der Trägerstruktur 40 entfernt ist, niedriger als der andere.

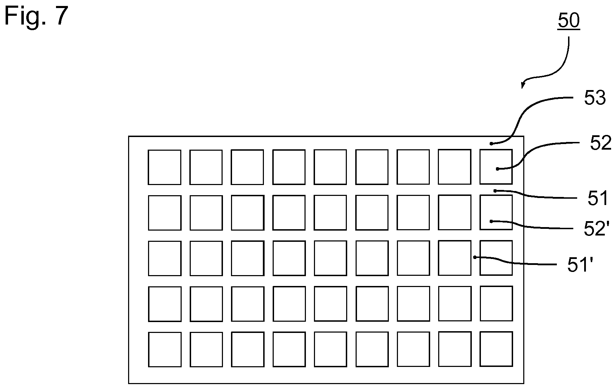

Fig. 7 zeigt schematisch eine Draufsicht auf eine Gussform 50, mit der sich beispielsweise die Neutralelektrodenvorrichtung 30 gemäß der Fig. 2 herstellen lässt. Diese weist einen im Wesentlichen rechteckigen Rahmen 53 auf, innerhalb dessen eine Vielzahl von Trennwänden 51, 51' in Quer- und Längsrichtung angeordnet sind. Die Trennwände 51, 51' begrenzen nach oben und unten hin offene Segmente 52, 52'. Ein erfindungsgemäßes Verfahren zur Herstellung einer Neutralelektrodenvorrichtung kann die folgenden Schritte umfassen:
- Herstellen einer Trägerstruktur 40;
- Auflegen der Gussform 50 auf die Trägerstruktur 40;
- Erwärmen von Phasenwechselmaterial derart, dass sich dieses zumindest teilweise verflüssigt;
- Eingießen des Phasenwechselmaterials in die Gussform 50 derart, dass die Segmente 52, 52' zumindest teilweise gefüllt sind und
- Entfernen der Gussform 50.

Vorzugsweise erfolgt das Entfernen nach einem zumindest teilweisen Aushärten der Blöcke 37, 37'. Gegebenenfalls können sich unterscheidende Phasenübergangsmaterialien - beispielsweise zur Herstellung des beschriebenen Schichtaufbaus - nacheinander eingebracht werden. Des Weiteren ist es denkbar, ein Mischverhältnis des PCMs beim Einbringen zu verwenden, das sich über die Zeit verändert, so dass beispielsweise der Schmelzpunkt des PCMs mit zunehmender Distanz zu der Trägerstruktur 40 abnimmt.

Weitere optionale Details zur möglichen Herstellung einer erfindungsgemäßen Trägerstruktur 40 mit PCM werden nachfolgend beschrieben.

Die einzelnen genannten und beschriebenen Ausführungsbeispiele lassen sich erfindungsgemäß in beliebiger Weise miteinander kombinieren. Beispielsweise kann auch bei dem Ausführungsbeispiel gemäß Fig. 4 ein Mehrschichtaufbau der PCM-Blöcke 37, 37' gewählt werden. Auch kann die Gussform 50 gemäß Fig. 7 derart abgewandelt sein, dass sich hiermit die Neutralelektrodenvorrichtung 30 gemäß Fig. 5 herstellen lässt. Des Weiteren ist beispielsweise auch bei der Neutralelektrodenvorrichtung 30 eine versetzte oder unregelmäßige Anordnung der PCM-Blöcke 37 denkbar. Auch können die PCM Blöcke 37 einer Neutralelektrodenvorrichtung 30 verschiedene Formen (Quader, Zylinder, Würfel, Trapez, Pyramide etc.) umfassen.

### Bezugszeichenliste

- 1: Torso
- 10: HF-Generator
- 20: monopolares Instrument
- 30: Neutralelektrodenvorrichtung
- 32: Trägervlies
- 33: PET-Träger
- 34, 34': Elektroden
- 36: Hydrogel
- 37, 37': PCM-Block
- 37a, 37b, 37c: PCM-Schicht
- 38, 38': Abstandsspalte
- 40: Trägerstruktur
- 50: Gussform
- 51, 51': Trennwand
- 52, 52': Segment
- 53: Rahmen

## Patentansprüche

1. Neutralelektrodenvorrichtung zur Applikation eines HF-Stroms an einem biologischen Gewebe, umfassend:
- eine Trägerstruktur (40) mit einer ersten und einer zweiten Seite;
- mindestens eine Elektrode (34, 34'), die an der ersten Seite der Trägerstruktur (40) angeordnet ist;
- ein Phasenwechselmaterial (PCM) zur Aufnahme von Wärme, das auf der zweiten Seite der Trägerstruktur (40) angeordnet ist,
**dadurch gekennzeichnet, dass**
das Phasenwechselmaterial zumindest teilweise als Blöcke (37, 37') ausgebildet und die Blöcke (37, 37') zur Ausbildung von Abstandsspalten (38, 38') voneinander zumindest teilweise beabstandet sind und wobei die Blöcke (37, 37') eine Polymerstruktur umfassen und die Polymerstruktur schwammartig ausgebildet ist, wobei das Phasenwechselmaterial in die Polymerstruktur verarbeitet ist.

2. Neutralelektrodenvorrichtung nach Anspruch 1,
**gekennzeichnet durch**
eine Vielzahl von Elektroden (34, 34') aus einer Aluminiumlegierung.

3. Neutralelektrodenvorrichtung nach einem der Ansprüche 1 oder 2,
**gekennzeichnet durch**
eine leitende Substanz aus der Gruppe der viskoelastischen Fluide, wobei die leitende Substanz auf der ersten Seite der Trägerstruktur (40) vorgesehen ist.

4. Neutralelektrodenvorrichtung nach einem der vorhergehenden Ansprüche,
**dadurch gekennzeichnet, dass**
die Blöcke (37, 37') als geometrische Körper, nämlich als Polyeder, ausgebildet sind.

5. Neutralelektrodenvorrichtung nach einem der vorhergehenden Ansprüche,
**dadurch gekennzeichnet, dass**
sich die Blöcke (37, 37') in Richtung von der Trägerstruktur weg verjüngen.

6. Neutralelektrodenvorrichtung nach einem der vorhergehenden Ansprüche,
**dadurch gekennzeichnet, dass**
die von den Blöcken (37, 37') begrenzten Abstandsspalten (38, 38') sich über die gesamte Trägerstruktur (40) entlang von Geraden erstrecken.

7. Neutralelektrodenvorrichtung nach einem der vorhergehenden Ansprüche,
**dadurch gekennzeichnet, dass**
eine Vielzahl der Blöcke (37, 37') eine identische Form hat und mit konstantem Abstand zueinander und/oder konstantem Versatz zueinander auf der Trägerstruktur (40) angeordnet ist.

8. Neutralelektrodenvorrichtung nach einem der vorhergehenden Ansprüche,
**dadurch gekennzeichnet, dass**
zumindest einige der Blöcke (37, 37') thermochrome Farbstoffe und/oder Farbpigmente umfassen.

9. Neutralelektrodenvorrichtung nach einem der vorhergehenden Ansprüche,
**dadurch gekennzeichnet, dass**
zumindest einige der Blöcke (37, 37') eine erste, untere Schicht (37a) mit einem ersten Phasenwechselmaterial und eine zweite, obere Schicht (37c) mit einem zweiten Phasenwechselmaterial umfassen, wobei das erste Phasenwechselmaterial einen Schmelzpunkt hat der deutlich, nämlich um mindestens 5%, höher ist als ein Schmelzpunkt des zweiten Phasenwechselmaterials.

10. Elektrochirurgisches System zur Koagulation und/oder zum Schneiden von Gewebe, umfassend:
- eine Neutralelektrodenvorrichtung (30) nach einem der vorhergehenden Ansprüche;
- mindestens ein elektrochirurgisches, insbesondere monopolares Instrument (20);
- einen HF-Generator, der mit der Neutralelektrodenvorrichtung (30) und dem mindestens einem Instrument (20) verbunden ist.

11. Verfahren zur Herstellung einer Neutralelektrodenvorrichtung, umfassend die Schritte:
- Herstellen einer Trägerstruktur (40)), wobei das Herstellen der Trägerstruktur (40) ein Aufbringen mindestens einer Elektrode (34, 34') auf eine Folie und ein Ankleben einer Faserstruktur auf der der Elektrode (34, 34') abgewandten Seite der Folie umfasst;
- Auflegen einer nach unten hin zumindest teilweise offenen Gussform (50) auf die Trägerstruktur (40), wobei die Gussform (50) eine Vielzahl von Segmenten (52, 52') zur Herstellung von Blöcken (37, 37') umfasst, die von benachbarten Segmenten (52, 52') durch Trennwände (51, 51') getrennt sind;
- Erwärmen eines Phasenwechselmaterials (PCM) derart, dass das Phasenwechselmaterial zumindest teilweise flüssig ist;
- Einbringen des erwärmten Phasenwechselmaterials in die Gussform (50) derart, dass ein Stoffschluss zwischen dem Phasenwechselmaterial und der Folie hergestellt wird;
- Entfernen der Gussform (50).

12. Verfahren nach Anspruch 11,
**dadurch gekennzeichnet, dass**
in die Gussform (50) nacheinander mehrere sich anhand des Schmelzpunkts unterscheidende Phasenwechselmaterialien eingebracht werden.

13. Verfahren nach einem der Ansprüche 11 oder 12,
**dadurch gekennzeichnet, dass**
mindestens ein Phasenwechselmaterial in einer schwammartigen, Polymerstruktur verarbeitet wird.

## Claims

1. Neutral electrode device for application of an RF current to a biological tissue, comprising:
- a supporting structure (40) having a first and a second side;
- at least one electrode (34, 34'), which is arranged on the first side of the supporting structure (40);
- a phase change material (PCM) for absorbing heat, which is arranged on the second side of the supporting structure (40),
**characterized in that**
the phase change material is formed at least in part as blocks (37, 37') and the blocks (37, 37') are arranged at least partially spaced apart from one another to form spacing gaps (38, 38') and wherein the blocks (37, 37') comprise a polymer structure and the polymer structure is formed in a sponge-like manner, the phase change material being worked into the polymer structure.

2. Neutral electrode device according to Claim 1,
**characterized by**
a multiplicity of electrodes (34, 34') of an aluminium alloy.

3. Neutral electrode device according to either of Claims 1 and 2,
**characterized by**
a conductive substance from the group of viscoelastic fluids, wherein the conductive substance is provided on the first side of the supporting structure (40).

4. Neutral electrode device according to one of the preceding claims,
**characterized in that**
the blocks (37, 37') are formed as geometric bodies, to be specific as polyhedrons.

5. Neutral electrode device according to one of the preceding claims,
**characterized in that**
the blocks (37, 37') taper in a direction away from the supporting structure.

6. Neutral electrode device according to one of the preceding claims,
**characterized in that**
the spacing gaps (38, 38') delimited by the blocks (37, 37') extend along straight lines over the entire supporting structure (40).

7. Neutral electrode device according to one of the preceding claims,
**characterized in that**
a multiplicity of the blocks (37, 37') have an identical form and are arranged on the supporting structure (40) at a constant distance from one another and/or constant offset from one another.

8. Neutral electrode device according to one of the preceding claims,
**characterized in that**
at least some of the blocks (37, 37') comprise thermochromic dyes and/or colour pigments.

9. Neutral electrode device according to one of the preceding claims,
**characterized in that**
at least some of the blocks (37, 37') comprise a first, lower layer (37a) having a first phase change material and a second, upper layer (37c) having a second phase change material, wherein the first phase change material has a melting point that is significantly higher, that is by at least 5%, than a melting point of the second phase change material.

10. Electrosurgical system for the coagulation and/or cutting of tissue, comprising:
- a neutral electrode device (30) according to one of the preceding claims;
- at least one electrosurgical, in particular monopolar instrument (20);
- an RF generator, which is connected to the neutral electrode device (30) and the at least one instrument (20).

11. Method for producing a neutral electrode device, comprising the steps of:
- producing a supporting structure (40), wherein producing the supporting structure (40) comprises applying at least one electrode (34, 34') to a film and adhesively attaching a fibre structure on the side of the film facing away from the electrode (34, 34');
- placing a casting mould (50), which is downwardly at least partially open, onto the supporting structure (40), wherein the casting mould (50) comprises a multiplicity of segments (52, 52') for producing blocks (37, 37'), which are separated from adjacent segments (52, 52') by partition walls (51, 51');
- heating a phase change material (PCM) in such a way that the phase change material is at least partially liquid;
- introducing the heated phase change material into the casting mould (50) in such a way that a mass transfer is established between the phase change material and the film;
- removing the casting mould (50).

12. Method according to Claim 11,
**characterized in that**
a number of phase change materials having different melting points are introduced one after the other into the casting mould (50).

13. Method according to either of Claims 11 and 12,
**characterized in that**
at least one phase change material is worked into a sponge-like polymer structure.

## Revendications

1. Dispositif d'électrode neutre destiné à l'application d'un courant HF à un tissu biologique, comprenant :
- une structure porteuse (40) ayant un premier et un deuxième côté ;
- au moins une électrode (34, 34') qui est disposée du premier côté de la structure porteuse (40) ;
- un matériau à changement de phase (PCM) destiné à absorber la chaleur, qui est disposé sur le deuxième côté de la structure porteuse (40),
**caractérisé en ce que**
le matériau à changement de phase est au moins partiellement réalisé sous la forme de blocs (37, 37') et les blocs (37, 37') sont au moins partiellement espacés les uns des autres en vue de former des intervalles d'écartement (38, 38') et les blocs (37, 37') comprenant une structure polymère et la structure polymère étant réalisée à la manière d'une éponge, le matériau à changement de phase étant façonné dans la structure polymère.

2. Dispositif d'électrode neutre selon la revendication 1, **caractérisé par** une pluralité d'électrodes (34, 34') en un alliage d'aluminium.

3. Dispositif d'électrode neutre selon l'une des revendications 1 et 2, **caractérisé par** une substance conductrice du groupe des fluides viscoélastiques, la substance conductrice se trouvant sur le premier côté de la structure porteuse (40).

4. Dispositif d'électrode neutre selon l'une des revendications précédentes, **caractérisé en ce que** les blocs (37, 37') sont réalisés sous la forme de corps géométriques, à savoir sous la forme de polyèdres.

5. Dispositif d'électrode neutre selon l'une des revendications précédentes, **caractérisé en ce que** les blocs (37, 37') se rétrécissent dans une direction s'éloignant de la structure porteuse.

6. Dispositif d'électrode neutre selon l'une des revendications précédentes, **caractérisé en ce que** les intervalles d'écartement (38, 38') délimités par les blocs (37, 37') s'étendent sur la totalité de la structure porteuse (40) le long de droites.

7. Dispositif d'électrode neutre selon l'une des revendications précédentes, **caractérisé en ce qu'**un grand nombre des blocs (37, 37') possèdent une forme identique et sont disposés sur la structure porteuse (40) avec un écart constant les uns des autres et/ou un décalage constant les uns par rapport aux autres.

8. Dispositif d'électrode neutre selon l'une des revendications précédentes, **caractérisé en ce qu'**au moins certains des blocs (37, 37') comprennent des colorants et/ou des pigments de couleur thermochromes.

9. Dispositif d'électrode neutre selon l'une des revendications précédentes, **caractérisé en ce qu'**au moins certains des blocs (37, 37') comprennent une première couche inférieure (37a) avec un premier matériau à changement de phase et une deuxième couche supérieure (37c) avec un deuxième matériau à changement de phase, le premier matériau à changement de phase ayant un point de fusion qui est nettement, à savoir au moins 5 % plus élevé qu'un point de fusion du deuxième matériau à changement de phase.

10. Système électrochirurgical destiné à la coagulation et/ou à la découpe de tissus, comprenant :
- un dispositif d'électrode neutre (30) selon l'une des revendications précédentes ;
- au moins un instrument (20) électrochirurgical, notamment monopolaire ;
- un générateur HF qui est relié au dispositif d'électrode neutre (30) et à l'au moins un instrument (20) .

11. Procédé de fabrication d'un dispositif d'électrode neutre, comprenant les étapes suivantes :
- fabrication d'une structure porteuse (40), la fabrication de la structure porteuse (40) comprenant une application d'au moins une électrode (34, 34') sur un film et un collage d'une structure de fibres sur le côté du film à l'opposé de l'électrode (34, 34') ;
- dépose d'un moule de coulée (50) au moins partiellement ouvert vers le bas sur la structure porteuse (40), le moule de coulée (50) comportant une pluralité de segments (52, 52') destinés à produire des blocs (37, 37') qui sont séparés des segments (52, 52') voisins par des parois de séparation (51, 51') ;
- chauffage d'un matériau à changement de phase (PCM) de telle sorte que le matériau à changement de phase est au moins partiellement liquide ;
- incorporation du matériau à changement de phase chauffé dans le moule de coulée (50) de telle sorte qu'une liaison par fusion de matières est créée entre le matériau à changement de phase et le film ;
- enlèvement du moule de coulée (50).

12. Procédé selon la revendication 11, **caractérisé en ce que** plusieurs matériaux à changement de phase qui se différencient par le point de fusion sont introduits les uns après les autres dans le moule de coulée (50).

13. Procédé selon l'une des revendications 11 et 12, **caractérisé en ce qu'**au moins un matériau à changement de phase est façonné en une structure polymère de type éponge.
